# EUROPEAN PATENT APPLICATION

(11) **EP 4 628 502 A1**
(43) Date of publication of application: **08.10.2025**
(21) Application number: 25165483.6
(22) Date of filing: 21.03.2025
(51) Int. Cl.: C07K 14/47, C12N 5/00, C12N 15/86

(54) **METHOD FOR CHANGING HLA PRESENTATION OF PEPTIDE IN CELL LINE, CELL LINE WITH CHANGED HLA PRESENTATION OF PEPTIDE AND USE THEREOF**

(30) Priority: 26.03.2024 US 202463569744 P; 13.03.2025 TW 114109294
(71) Applicant: Acer Incorporated, New Taipei City 221 (TW)
(72) Inventor: CHANG, Ruei-Chuan, Taipei City (TW); YEH, Li-Tzu, Taipei City (TW); CHIU, Chih-Wei, Taipei City (TW); TSAI, Tsung-Hsien, Taipei City (TW)
(74) Representative: Haseltine Lake Kempner LLP

(57) **Abstract**

The invention provides a method for changing an HLA presentation of peptides in a cell line, a cell line with a changed HLA presentation of peptides and a use thereof. The method for changing the HLA presentation includes following steps. HLA genes are lentiviral-transduced into K562 cell lines, so that the cell lines express HLA molecules. Afterwards, the cell lines expressing the HLA molecule are transduced with a lentiviral vector carrying an AIRE gene. Through the overexpression of AIRE, a gene transcription profile and a protein profile expressed by the cell line are changed, resulting in a change in a type of peptide presented by the HLA molecule.

## Description

### BACKGROUND

### Technical Field

The invention relates to a method for changing an HLA presentation of peptides in a cell line, a cell line with a changed HLA presentation of peptides and a use thereof.

### Description of Related Art

Since the effectiveness of AI models is highly dependent on the diversity and representativeness of training data, when the training data is insufficient to cover all possible biological variability, the generalization ability of the model will be limited. The invention ectopically expresses the autoimmune regulator (AIRE) gene to change the cellular gene transcription profile and protein profile, thereby increasing the diversity of HLA-presented peptides. AIRE is expressed in the thymic medulla and plays an important role in the negative selection of immune T cells during thymic development. The T cell receptor (TCR) on the surface of each T cell recognizes a specific antigen presented by the HLA (Human Leukocyte Antigen) complex on an antigen-presenting cell. TCR is produced by VDJ recombination fragments and has high diversity and antigen specificity. Some T cells recognize self-antigens presented by HLA and induce autoimmune diseases. These T cells must be eliminated through negative selection in the thymus during development. Through the AIRE regulation, medullary thymic epithelial cells (mTECs) express tissue-specific proteins of various tissue cells in the body. When the TCR of T cells that may cause autoimmune diseases has a strong binding force with the HLA molecule complex on the surface of mTECs, it will induce apoptosis of T cells that may cause autoimmune diseases.

Cell lines lacking HLA expression (e.g., K562 cell line, a human chronic myeloid leukemia cell line) can be used for HLA-related cellular immunology studies. AIRE is a transcription factor that can induce the expression of tissue-specific proteins that were originally only expressed in specific cells.

### SUMMARY

The invention provides a method for changing an HLA presentation of peptides in a cell line, a cell line with a changed HLA presentation of peptides and a use thereof. Through the effect of AIRE, K562-HLA cell lines will express tissue-specific proteins that are only expressed in other tissues of the body, increasing the chances of HLA presenting different peptide sequences.

The method for changing the HLA presentation of peptides in a cell line of the invention includes following steps. HLA genes are lentiviral-transduced into cell lines, so that the cell lines express HLA molecules. Afterwards, the cell lines expressing the HLA molecule are transduced with a lentiviral vector carrying an AIRE gene. Through the overexpression of AIRE, a gene transcription profile and a protein profile expressed by the cell line are changed, resulting in a change in a type of peptide presented by the HLA molecule.

In an embodiment of the invention, the cell line includes a human chronic myeloid leukemia cell line.

In an embodiment of the invention, the human chronic myeloid leukemia cell line includes a K562 cell line.

In an embodiment of the invention, the HLA molecules include HLA-A molecules, HLA-B molecules or HLA-C molecules.

A cell line with a changed HLA presentation of peptides of the invention is prepared by the aforementioned method.

A use of a cell line of the invention, including: after extracting proteins from the cell line, HLA molecules and bound peptides thereof are separated from the proteins of the cell line by an immunoprecipitation method, and peptides presented by a single HLA are obtained by a LC-MS/MS analysis and a data analysis; a RNA analysis is performed on the cell line by next generation sequencing (NGS); and the peptides presented by the single HLA and the RNA analysis are used as data required for establishing an artificial intelligence model, and the artificial intelligence model is used to predict peptides presented by HLA molecules.

Based on the above, the invention provides a method for changing an HLA presentation of peptides in a cell line, a cell line with a changed HLA presentation of peptides and a use thereof. The HLA gene is transduced into a cell line lacking HLA molecule expression by lentivirus, causing it to express HLA molecules. Through the effect of AIRE, the tissue-specific proteins that were originally only expressed in specific cells are induced to express, thereby changing the total type and expression amount of proteins expressed by the cell line, further causing changes in the types of peptides presented by HLA, so as to improve the performance of the artificial intelligence model.

### DESCRIPTION OF THE EMBODIMENTS

The following examples are described in detail with reference to the accompanying drawings, but the provided embodiment s are not intended to limit the scope covered by this disclosure. Furthermore, terms such as "contain", "include", "have", etc. used in the text are open-ended terms, meaning "including but not limited to".

The invention proves a method for changing the HLA presentation of peptides in a cell line, including the following steps. HLA genes are lentiviral-transduced into cell lines lacking HLA molecule expression, so that the cell lines express HLA molecules. Afterwards, the cell lines expressing the HLA molecule are transduced with a lentiviral vector carrying an AIRE gene. Through the overexpression of AIRE, a gene transcription profile and a total type and an expression amount of proteins expressed by the cell line are changed, resulting in a change in a type of peptide presented by the HLA molecule.

In this embodiment, the cell line may include a human chronic myeloid leukemia cell line, such as a K562 cell line, but the invention is not limited thereto, and other human cell lines that can be cultured in a laboratory may also be used, such as Expi293, HEK293, A375, KG-1, or B721.221 cell lines. The HLA molecule may include an HLA-A molecule, an HLA-B molecule, or an HLA-C molecule.

In this embodiment, the method of transducing HLA genes into cell lines lacking HLA molecule expression is to transduce with lentiviral vectors, culture them in a cell culture medium containing puromycin after infection, and remove uninfected cells.

In this embodiment, the cell line expressing HLA molecules is infected with a lentivirus carrying the AIRE gene. After the cell line carrying HLA and AIRE molecules is established, it is cultured in a cell culture medium containing blasticidin S and the uninfected cells are removed.

In this embodiment, by ectopically expressing AIRE, the gene transcription profile of the cell line is changed. The results of RNAseq analysis shows that 648 genes increased by more than 2 times, and 288 genes decreased by more than 2 times. Proteomic analysis reveals 606 additional proteins in cells expressing AIRE. That is, AIRE is used to change the gene transcription and protein expression patterns in the original cell line.

The invention provides a cell line with a changed HLA presentation of peptides, which is prepared by the above method for changing an HLA presentation of peptides in a cell line. In this embodiment, the cell line may include a human chronic myeloid leukemia cell line, such as a K562 cell line, but the invention is not limited thereto, and other human cell lines that can be subcultured in a laboratory may also be used. The HLA molecule may include an HLA-A molecule, an HLA-B molecule, or an HLA-C molecule. The results of HLA-presented peptide analysis shows that HLA-A, B, and C molecules in cells expressing AIRE presented 2,141, 1,613, and 447 new peptide sequences, respectively.

The invention provides a use of a cell line, which is the above cell line with a changed HLA presentation of peptides, and includes the following contents. After extracting proteins from the cell line, HLA molecules and bound peptides thereof are separated from the proteins of the cell line by an immunoprecipitation method, and peptides presented by a single HLA are obtained by a LC-MS/MS analysis and a data analysis; a RNA analysis is performed on the cell line by next generation sequencing (NGS); and the peptides presented by the single HLA and the RNA analysis are used as data required for establishing an artificial intelligence model, and the artificial intelligence model is used to predict peptides presented by HLA molecules.

In this embodiment, when a RNA analysis is performed on K562 cell lines with and without ectopic AIRE expression by next-generation sequencing (NGS), the results shows that the RNA expression of 648 genes is significantly increased in the cell lines with ectopic AIRE expression.

The following experimental examples are used to illustrate in detail that a cell line with a changed HLA presentation of peptides of the invention are helpful in improving the performance of artificial intelligence models. However, the following experimental examples are not intended to limit the invention.

### Experimental Examples

### Experimental Example 1-A0201

| | Overlap testing set ( 1:999 / n=437 ) | |
|---|---|---|
| Data source (number of training sets) | TopN PPV | Recall N PPV |
| Ref (1500) | 0.403 | 0.408 |
| Ref+AIRE (3000) | 0.453 | 0.497 |
| Ref large (3024) | 0.414 | 0.424 |

### Experimental Example 2-B1301

For the experimental results of Experimental Example 2, please refer to Table 1 below.

**Table 1**

| | Overlap testing set ( 1:999 / n=740 ) | |
|---|---|---|
| Data source (number of training sets) | TopN PPV | Recall N PPV |
| Ref (2000) | 0.364 | 0.334 |
| Ref + AIRE (2000+1000) | 0.420 | 0.437 |
| Ref large (3702) | 0.384 | 0.375 |

### Experimental Example 3- C1601

For the experimental results of Experimental Example 3, please refer to Table 2 below.

**Table 2**

| | Overlap testing set ( 1:999 / n=740 ) | |
|---|---|---|
| Data source (number of training sets) | TopN PPV | Recall N PPV |
| Ref (2000) | 0.354 | 0.296 |
| Ref + AIRE (2000+1000) | 0.458 | 0.503 |
| Ref large (2725) | 0.391 | 0.383 |

From the above experimental examples, it can be seen that through the effect of AIRE, the total types and expression levels of RNA and proteins expressed by the cell line are changed, which further causes changes in the types of peptides presented by HLA, increases the chances of HLA presenting different peptide sequences, and helps to improve the performance of artificial intelligence models.

In summary, the invention provides a method for changing an HLA presentation of peptides in a cell line, a cell line with a changed HLA presentation of peptides and a use thereof, wherein the HLA gene is transduced into a cell line lacking HLA molecule expression by lentivirus to express HLA molecules, and then through the effect of AIRE, tissue-specific proteins that were originally only expressed in specific cells are induced to be expressed, thereby changing the total type and expression amount of proteins expressed by the cell line, further causing changes in the types of peptides presented by HLA, increasing the chances of HLA presenting different peptide sequences, and thus helping to improve the performance of artificial intelligence models.

## Claims

1. A method for changing an HLA presentation of peptides in a cell line, comprising:
lentiviral-transducing HLA genes into a cell line lacking an HLA molecule expression, so that the cell line expresses HLA molecules; and
transducing the cell line expressing the HLA molecules with a lentivirus vector carrying an AIRE gene, and an overexpression of AIRE changes a gene transcription profile, a protein type and expression levels expressed by the cell line, resulting in a change in a type of peptide presented by the HLA molecule.

2. The method according to claim 1, wherein the cell line includes a human chronic myeloid leukemia cell line.

3. The method according to claim 2, wherein the human chronic myeloid leukemia cell line includes a K562 cell line.

4. The method according to claim 1, wherein the HLA molecules include HLA-A molecules, HLA-B molecules or HLA-C molecules.

5. A cell line with a changed HLA presentation of peptides, which is prepared by the method according to any of claims 1 to 4.

6. A use of a cell line, which is the cell line according to claim 5, comprising:
after extracting proteins from the cell line, separating HLA molecules and bound peptides thereof from the proteins of the cell line by an immunoprecipitation method, and obtaining peptides presented by a single HLA by a LC-MS/MS analysis and a data analysis;
performing a RNA analysis on the cell line by next generation sequencing (NGS); and
using the peptides presented by the single HLA and the RNA analysis as data required for establishing an artificial intelligence model, and the artificial intelligence model is used to predict peptides presented by HLA molecules.
